Europäisches Patentamt

European Patent Office

Office européen des brevets

(19)

(11) Veröffentlichungsnummer: **0 212 517**
A2

# (12) EUROPÄISCHE PATENTANMELDUNG

(21) Anmeldenummer: 86111036.9

(22) Anmeldetag: 09.08.86

(51) Int. Cl.⁴: **C 12 P 7/18**, C 12 P 7/06,
C 12 P 7/58
// (C12P7/18,
C12R1:01),(C12P7/06,
C12R1:01),(C12P7/56,
C12R1:01)

(30) Priorität: 13.08.85 DE 3528932

(43) Veröffentlichungstag der Anmeldung: 04.03.87
Patentblatt 87/10

(84) Benannte Vertragsstaaten: AT BE DE FR GB IT NL SE

(71) Anmelder: Kernforschungsanlage Jülich Gesellschaft mit beschränkter Haftung, Postfach 1913, D-5170 Jülich 1 (DE)

(72) Erfinder: Bringer-Meyer, Stephanie, Dr., Buchheimer Strasse 23, D-4150 Krefeld-Bockum (DE)
Erfinder: Sahm, Hermann, Prof., Wendelinusstrasse 71, D-5170 Jülich (DE)

(54) **Verfahren zur Gewinnung von Sorbit durch enzymatische Reaktion und dafür geeignete Mikroorganismen.**

(57) Ausgehend von Glucose und Fructose enthaltenden wäßrigen Mischungen wird durch Fermentation mittels einer Fructose nicht vergärenden stabilen Mutanten von Zymomonas mobilis eine wäßrige Mischung von Fructose, Sorbit, Glucose, Ethanol, $CO_2$ und Gluconsäure erhalten, aus welcher der Sorbit isoliert werden kann. Die Ausgangsmischungen enthalten zumindest je 100 g/l, vorzugsweise je 120 bis 200 g/l und speziell je 140 bis 180 g/l Glucose und Fructose. Die Mutante mit der Hinterlegungsnummer DSM 3126 hat sich als geeignet erwiesen.

EP 0 212 517 A2

Kernforschungsanlage Jülich
Gesellschaft mit beschränkter Haftung

0212517

Verfahren zur Gewinnung von Sorbit durch enzymatische Reaktion und dafür geeignete Mikroorganismen

---

Die Erfindung bezieht sich auf ein Verfahren zur Gewinnung von Sorbit durch enzymatische Reaktion ausgehend von Glucose und Fructose enthaltenden wässrigen Mischungen sowie auf dafür geeignete Mutanten von Zymomonas mobilis.

Sorbit hat ca. 50 - 60 % der Süßkraft von Saccharose und wird wegen seiner günstigen physiologischen und anwendungstechnischen Eigenschaften in großem Umfang in der Kosmetik, der Pharmazie, der Lebensmittelindustrie und auf dem technischen Sektor verwendet. Da Sorbit im Organismus insulinsparend verwertet wird, wird er insbesondere als Zuckeraustauschstoff für Diabetiker eingesetzt.

Bei den heute gebräulichen Verfahren der Sorbitherstellung werden wäßrige Glucoselösungen bei 120 - 150 °C und $H_2$-Drucken von 70 - 180 bar in Gegenwart von Schwermetall-Katalysatoren (z.B. Raney-Nickel) hydriert und die Produktlösungen durch einen Mehrstufen-Prozess über Filtration, Ionenaustausch und Aktivkohle-Behandlung gereinigt. Da der Reinheitsgrad des Sorbits von dem des Ausgangsmaterials abhängt, werden hochreine Glucoselösungen für die Sorbitproduktion eingesetzt. Hohe Substrat- und Prozesskosten, vor

...

allem Energiekosten für hohe Drucke und Temperaturen machen diese chemischen Verfahren der Sorbitherstellung relativ aufwendig und teuer. Es wurden daher auch bereits Versuche unternommen, Sorbit bei Normaldruck und niedrigen Temperaturen mittels biologischer Systeme herzustellen.

So wird in der DE-OS 33 26 546 ein Verfahren zur kontinuierlichen enzymatischen Herstellung von Gluconsäure sowie Sorbit und Mannit aus Glucose und Fructose, bzw. Glucose und/oder Fructose enthaltenden Disacchariden beschrieben. In einem Membranreaktor wird dabei durch das Enzym Glucose-Dehydrogenase Glucose zu Gluconsäure oxidiert. Die im Substratgemisch enthaltene Fructose kann durch eine parallele Reaktion wahlweise mit dem Enzym Mannit-Dehydrogenase zu Mannit oder mit dem Enzym Sorbit-Dehydrogenase zu Sorbit reduziert werden. Für die Glucoseoxidation (1) und die Fructosereduktion (2) ist außerdem das Coenzym NAD(H) zwingend notwendig, welches in Reaktion (1) Akzeptor und in Reaktion (2) Donator eines Hydrid-Ions ist. Da NAD(H) wegen seines niedrigen Molekulargewichtes nicht in dem verfahrensgemäßen Membranreaktor zurückgehalten werden kann, muß das selbst schon kostspielige Coenzym noch mit einem wasserlöslichen Polymer immobilisiert werden. Dieses Verfahren macht also die Reinigung mehrerer Enzyme sowie die Synthese polymergebundenen Cofaktors notwendig. Technisch erscheint so das Verfahren nach der DE-OS 33 26 546 als zu aufwendig für die Herstellung von Sorbit.

...

Die JP - A1 - 82/19 00 53 beschreibt ein Verfahren, bei dem durch zellfreie Extrakte der Hefen Pichia guercuum oder Pichia xylosa aus Glucose in Gegenwart von NADPH Sorbit gewonnen wird. Bei diesem Verfahren sind erhebliche Nachteile in der Vorkultivierung und Aufarbeitung der Hefezellen, sowie in dem kostspieligen Einsatz von NADPH zu sehen, so daß der Aufwand - insbesondere im Vergleich zu den sehr niedrigen Ausbeuten von 0,006 bzw. 0,020 mol Sorbit/mol Glucose - unverhältnismäßig hoch ist.

Die Verwendung gereinigter oder ungereinigter Enzympräparate und der Cofaktoren NAD(H) oder NADPH macht somit die bekannten biologischen Verfahren der Sorbitgewinnung ebenfalls aufwendig und so kostspielig, daß sie in der Praxis bisher kaum Einsatz finden.

Ziel der Erfindung ist daher ein Verfahren zur Herstellung von Sorbit auf relativ einfache und wenig kostspielige Weise.

Dieses Ziel wird gemäß der Erfindung bei dem eingangs genannten Verfahren dadurch erreicht, daß die wässrige Mischung unmittelbar mittels Fructose nicht vergärender stabiler Mutanten von Zymomonas mobilis fermentiert wird unter Bildung einer Fructose, Sorbit, Glucose, Ethanol, $CO_2$ und Gluconsäure enthaltenden wässrigen Mischung, aus welcher der Sorbit ggfs. isoliert wird.

...

Es wurde nämlich überraschend gefunden, daß Sorbit in relativ hoher Ausbeute aus wässrigen Mischungen erhalten werden kann, die sowohl Glucose als auch Fructose enthalten, wenn man die Vergärung von Glucose zu Ethanol mit stabilen fructosenegativen Mutanten von Zymomomas mobilis durchführt, die mindestens 10 Tage vorzugsweise mindestens 100 Tage lang die Fructosenegativität beibehalten.

Diese günstige Verfahrensweise hat außer der einfachen und wenig kostspieligen Durchführbarkeit den besonderen Vorteil, daß keine Zugabe von NAD (P) (H)-Cofaktoren erforderlich ist.

Aus einem Aufsatz von L. Viikari (Appl. Microbiol. & Biotechn. 20 (1984) 118-123) ist zwar bekannt, daß Sorbit ausgehend von wäßrigen Mischungen von Glucose und Fructose durch Äthanol-Fermentation mittels eines Wildstamms von Zymomonas mobilis erhalten werden kann, jedoch wird dabei die vorhandene Fructose vornehmlich zu Äthanol und $CO_2$ umgesetzt und die Sorbit-Ausbeute ist relativ gering. Erst wenn eine stabile Mutante von Zymomonas mobilis in der nun entwickelten Art angewandt wird, kann Sorbit fortlaufend wirtschaftlich erfolgreich neben Fructose unter Fermentation der Glucose produziert werden.

Vorzugsweise erfolgt die Umsetzung ausgehend von wässrigen Mischungen, die mindestens je 100g/l, vorzugsweise je 120 bis 200 g/l und insbesondere

...

je 140 bis 180 g/l Glucose und Fructose enthalten, wobei zweckmäßigerweise entweder batch-weise oder auch kontinuierlich mit Biomasse-Rückhaltung oder -Rückführung gearbeitet wird.

Für die Durchführung des erfindungsgemäßen Verfahrens geeignet sind spezielle neue stabile Mutanten von Zymomonas mobilis, die durch Mutation eines Wildstammes von Zymomonas mobilis durch physikalische und/oder chemische mutagene Mittel, Isolierung der sorbitbildenden fructosenegativen Mutanten und Selektion der stabilen Mutanten mittels kontinuierlicher Kultur über zumindest 10 Tage erhältlich sind und die Eigenheit besitzen, daß sie in Glucose/Fructose-Mischungen aus Fructose Sorbit bilden und nur aus Glucose nicht aber aus Fructose Ethanol, $CO_2$ und Gluconsäure produzieren.

In der Praxis hat sich die bei der Deutschen Sammlung von Mikroorganismen in Göttingen hinterlegte Mutante mit der Hinterlegungsnummer DSM 3126 als besonders zweckmäßig erwiesen.

Aus der erhaltenen Reaktionsmischung kann der Sorbit in bekannter Weise, z.B. durch Chromatografie oder fraktionierte Fällungen isoliert werden.

Nach dem erfindungsgemäßen Verfahren wird, insbesondere bei höheren Konzentrationen der Ausgangslösung, aus Fructose das Reduktionsprodukt Sorbit gebildet. Verlängerte Reaktionszeiten, die durch

...

verlangsamtes Wachstum von Zymomonas mobilis in Gegenwart solch hoher Zuckerkonzentrationen verursacht werden, können erfindungsgemäß durch den Einsatz kontinuierlicher Fermentationsmethoden mit Biomasse-Rückführung oder -Rückhaltung vermieden werden. Dabei kann die Durchflußrate der Substratlösung durch das Fermentergefäß vorzugsweise so eingestellt werden, daß Sorbit in hohen Ausbeuten erhalten wird. Günstig sind bei hohen Zelldichten im Fermenter hohe Zuckerkonzentrationen und Durchflußraten, bei denen noch Glucosereste im Ablauf verbleiben.

Das nicht reduzierte Ausgangsprodukt Fructose muß bei vielen Anwendungen nicht abgetrennt werden. Die ggfs. erwünschte Abtrennung der nicht umgesetzten Zucker kann nach bekannten Verfahren erfolgen. Ein besonderer Vorteil des erfindungsgemäßen Verfahrens ist die Möglichkeit der gezielten Einstellung verschiedener Sorbit/Fructose-Verhältnisse über die gewählten Konzentrationen der Ausgangslösungen.

Für die Biomasse-Rückhaltung eignen sich Immobilisierungen mit Alginaten oder $\kappa$-Carrageenanen, für die Biomasse-Rückführung by-pass Filtrationen des Fermenterauslaufs durch bakteriendichte Filter oder Hohlfaser-Module oder Sedimentationen bzw. Zentrifugieren.

Die Induktion der erfindungsgemäßen Mutanten in Wildstämmen von Zymomonas mobilis kann durch Be-

...

strahlung der Zellen mit ultraviolettem Licht, vorzugsweise durch chemische Mutagenese mit nicht-alkylierenden Agentien wie Nitrit, Bisulfit, Hydroxylamin oder mit alkylierenden Agentien wie Alkylalkansulfonate (z.B. Ethylmethansulfonat, Ethylethansulfonat, Diethylsulfat) und N-Nitroso-Verbindungen (z.B. Dialkyl-Nitrosamine, N-Nitrosoharnstoffe, N-Alkyl-N'-nitro-N-nitrosoguanidine), besonders bevorzugt durch das alkylierende Agenz N-Methyl-N'-nitro-N-nitrosoguanidin erfolgen.

Für die Kultivierung der erfindungsgemäßen Mutanten eignen sich Medien, die die folgenden Bestandteile enthalten (Mengen in g/l $H_2O$): $KH_2PO_4$: 0,5-1,0; $(NH_4)_2SO_4$: 0,5-1,0; $MgSO_4$ x $7H_2O$: 0,5-0,75; Hefeextrakt: 5-10; vergärbaren Zucker (Glucose): 10-130; der pH-Wert des Mediums sollte zu Beginn und während der Fermentation zwischen 4 und 7 liegen, die Temperatur zwischen 25 ° und 37 °C, vorzugsweise bei 28 bis 32 °C.

Weitere Besonderheiten gehen aus der nachfolgenden Beschreibung der Erfindung anhand von Beispielen hervor.

1) Induktion und Selektion fructose-negativer stabiler Mutanten

Eine Zellsuspension des Wildstammes Zymomonas mobilis ATCC 29191 wurde mit einer

...

Zelldichte von $2,8 \times 10^8$ Lebendzellen/ml, entsprechend einer optischen Dichte ($OD_{550\ nm}$) von 7,0 in 0,1 M Kaliumphosphatpuffer, pH 6,0 mit $1,9 \times 10^{-4}$ M N-Methyl-N´-nitro-N-nitrosoguanidin (NTG) suspendiert. Nach 15-minütiger Inkubation bei Raumtemperatur wurden die Zellen zweimal mit 0,9 % NaCl-Lösung gewaschen. Zur Mutations-Ausprägung folgte eine 20-stündige Inkubation in Flüssigmedium (g/l $H_2O$: $KH_2PO_4$, 1,0; $(NH_4)_2SO_4$, 1,0; $MgSO_4$ x $7H_2O$, 0,5; Hefeextrakt, 5; pH = 5,0) mit 2 % Glucose bei 30 °C. Eine anschließende Inkubation in Flüssigmedium mit 2 % Fructose + 0,3 mg/ml Ampicillin diente der Mutantenanreicherung. Diese Ausprägungs- und Anreicherungsphasen wurden dreimal wiederholt. Fructose-negative Mutanten wurden durch Replikaplattierungen auf Glucose bzw. Fructose enthaltenden Agarplatten (Flüssigmedium + 2 % Agar) identifiziert und isoliert.

Die Stabilität der fructose-negativen Mutanten wurde durch Ausplattierung unverdünnter Kulturproben auf Fructose enthaltenden Agarplatten (Flüssigmedium + 2 % Agar) überprüft. Kulturen der Mutante DSM 3126 blieben während und nach einem Zeitraum von 6 Monaten, bei 2-7-tägigen Überimpfungszeiträumen, in Flüssigkultur stabil. Ebenso blieb die Fructose-Negativität dieser Mutante während des Wachstums in kontinuierlicher Kultur über 5 Tage unverändert erhalten.

...

2) Gewinnung von Sorbit

Beispiel 1

Ein Medium bestehend aus 148,0 g/l Fructose und 151,0 g/l Glucose, gelöst in dem unter 1) beschriebenen Flüssigmedium wurde in einem pH- und Temperatur-regulierten Fermenter mit Zellen einer Vorkultur (5 g Zellfeuchtmasse/l) der Mutante Zymomonas mobilis DSM 3126 beimpft. Vom Zeitpunkt des Animpfens bis zum Ende des Fermenterlaufs wurde der pH-Wert auf 5,0 und die Temperatur bei 30 °C gehalten. Die Ergebnisse sind in Tabelle 1 angegeben.

Beispiel 2

Die Fermentation wurde wie in Beispiel 1 durchgeführt, jedoch mit höheren Zuckerkonzentrationen. Die Ergebnisse sind in Tabelle 1 zusammengestellt.

Tabelle 1

|  | Beispiel 1 | Beispiel 2 |
| --- | --- | --- |
| Anfangskonzentrationen: | | |
| Fructose (g/l) | 148,0 | 176,4 |
| Glucose (g/l) | 151,0 | 175,5 |
| Endkonzentrationen: | | |
| Sorbit (g/l) | 65,3 | 93,3 |
| Fructose (g/l) | 76,9 | 75,5 |
| Glucose (g/l) | 14,7 | 23,3 |
| Ethanol (g/l) | 54,6 | 54,0 |
| Fermentationszeit (h) | 75 | 110 |

Wie man sieht, ist die Sorbitbildung bei erhöhten Zuckerkonzentrationen und längeren Gärzeiten vermehrt.

0212517

Kernforschungsanlage Jülich
Gesellschaft mit beschränkter Haftung


P a t e n t a n s p r ü c h e


1. Verfahren zur Gewinnung von Sorbit durch enzymatische Reaktion ausgehend von Glucose und Fructose enthaltenden wässrigen Mischungen, d a - d u r c h   g e k e n n z e i c h n e t, daß die wässrige Mischung unmittelbar mittels Fructose nicht vergärender stabiler Mutanten von Zymomonas mobilis fermentiert wird unter Bildung einer Fructose, Sorbit, Glucose, Ethanol, $CO_2$ und Gluconsäure enthaltenden wässrigen Mischung, aus welcher der Sorbit ggfs. isoliert wird.

2. Verfahren nach Anspruch 1, d a d u r c h   g e k e n n z e i c h n e t, daß die Zuckerkonzentrationen der wäßrigen Mischungen mindestens je 100 g/l, vorzugsweise je 120 bis 200 g/l und besonders bevorzugt je 140 bis 180 g/l Glucose und Fructose betragen.

3. Verfahren nach Anspruch 1 oder 2, d a d u r c h   g e k e n n z e i c h n e t, daß die Fermentation batch-weise oder insbesondere kontinuierlich mit Biomasse-Rückhaltung oder -Rückführung durchgeführt wird.

4. Sorbit bildende stabile fructosenegative Mutanten von Zymomonas mobilis erhältlich durch Mutation eines Wildstammes von Zymomonas mobilis durch physikalische und/oder chemische mutagene Mittel,

PT    1.785
      nö/Fr

Isolierung sorbitbildender fructosenegativer Mutanten und Selektion der stabilen Mutanten mittels kontinuierlicher Kultur über zumindest 10 Tage.

5. Mutante von Zymomonas mobilis nach Anspruch 4, gekennzeichnet durch die Hinterlegungsnummer DSM 3126.